# EUROPEAN PATENT SPECIFICATION

(11) **EP 3 307 238 B1**
(45) Date of publication and mention of the grant of the patent: **31.12.2025**
(21) Application number: 16731753.6
(22) Date of filing: 13.06.2016
(51) Int. Cl.: A61K 38/46, A61K 9/00, A61K 9/08, A61K 47/02, A61P 27/02, A61P 31/12, A61P 31/20, A61P 31/22

(54) **PHARMACEUTICALS FOR TREATMENT OF VIRAL INFECTIONS OF THE EYE**
ARZNEIMITTEL ZUR BEHANDLUNG VON VIRUSINFEKTIONEN DES AUGES
PRODUITS PHARMACEUTIQUES PUR LE TRAITEMENT DES INFECTIONS VIRALES DE L'OEIL

(30) Priority: 15.06.2015 US 201562175961 P
(43) Date of publication of application: 18.04.2018
(73) Proprietor: Orgenesis Inc., Germantown, Maryland 20876 (US)
(72) Inventor: SQUIQUERA, Luis, Buenos Aires, 1425 (AR); SULLEY, Jamie, La Jolla, CA 92037 (US)
(74) Representative: Pearl Cohen Zedek Latzer Baratz UK LLP
(86) International application number: PCT/US2016/037174
(87) International publication number: WO 2016/205109

(56) References cited:
- WO-A1-2013/039857
- WO-A2-2015/148768
- US-A1- 2012 121 569
- US-A1- 2012 149 085
- US-A1- 2015 010 524
- REBECCA F. TURCOTTE ET AL: "Design and Characterization of an HIV-Specific Ribonuclease Zymogen", AIDS RESEARCH AND HUMAN RETROVIRUSES, vol. 24, no. 11, 1 November 2008 (2008-11-01), pages 1357 - 1363, XP055015162, ISSN: 0889-2229, DOI: 10.1089/aid.2008.0146
- S. K. SAXENA ET AL: "Inhibition of HIV-1 Production and Selective Degradation of Viral RNA by an Amphibian Ribonuclease", JOURNAL OF BIOLOGICAL CHEMISTRY, vol. 271, no. 34, 23 August 1996 (1996-08-23), pages 20783 - 20788, XP055202278, ISSN: 0021-9258, DOI: 10.1074/jbc.271.34.20783
- ILINSKAYA O N ET AL: "Ribonucleases as antiviral agents", MOLECULAR BIOLOGY : COVER-TO-COVER TRANSLATION = MOLEKULYARNAYA BIOLOGIYA, ACADEMY OF SCIENCES OF THE USSR, RU, vol. 48, no. 5, 11 October 2014 (2014-10-11), pages 615 - 623, XP035408512, ISSN: 0026-8933, [retrieved on 20141011], DOI: 10.1134/S0026893314040050
- KIMBERLY D DYER ET AL: "The RNase a superfamily: Generation of diversity and innate host defense", MOLECULAR DIVERSITY, KLUWER ACADEMIC PUBLISHERS, DO, vol. 10, no. 4, 13 September 2006 (2006-09-13), pages 585 - 597, XP019452837, ISSN: 1573-501X, DOI: 10.1007/S11030-006-9028-2

## Description

### Background of the Invention

The invention relates to viral infections of the eye, and more particularly relates to pharmaceuticals for treatment of viral infections of the eye. In its most immediate sense, the invention relates to treatment of human eye infections caused by *Adenoviridae* viruses.

Viral diseases of the eye can have significant consequences. Type 1 *herpes simplex virus* can cause conjunctivitis and keratitis, Human cytomegalovirus can cause retinitis, *adenovirus* types 8, 19, 29, and 37 can cause epidemic keratoconjunctivitis, and *adenovirus* types 3, 4, and 7 can cause pharyngoconjuctival fever. Herpes zoster virus (HZV), a member of the *Herpesviridae* family, can cause severe eye disease when affecting the trigeminal area. Herpes zoster ophthalmicus, a severe form of acute *herpes zoster,* results from the reactivation of *varicella zoster* virus (VZV) (another member of the *Herpesviridae* family) in the trigeminal (fifth cranial) nerve. Any branch of the nerve may be affected, though the frontal branch within the first division of the trigeminal nerve is most commonly involved. This frontal branch innervates nearly all of the ocular and periocular structures. Herpes zoster ophthalmicus at this particular location can lead to blindness and requires a fast and effective therapeutic approach.

*Human cytomegalovirus* (CMV) is another member of the *Herpesviridae* family. At least 60% of the US population has been exposed to CMV, with a prevalence of more than 90% in high-risk groups (e.g., unborn babies whose mothers become infected with CMV during pregnancy, people with HIV, and transplant recipients).

CMV retinitis is one of the most common opportunistic infections in persons with AIDS or pharmacologically induced immunosuppression. Individuals with CMV retinitis typically exhibit a progressive decrease in visual acuity, which may progress to blindness. Long-term CMV treatment is necessary to prevent retinitis relapse.

Immune reconstitution syndrome (IRIS) is reported in 16%-63% of HIV- infected patients with CMV retinitis following the initiation of HAART (Highly Active Antiretroviral Treatment). CMV IRIS may manifest as painless floaters, blurred vision, photopia, decreased visual acuity, or ocular pain. Some patients may develop macular edema leading to vision loss or proliferative vitreoretinopathy, spontaneous vitreal hemorrhage, and retinal detachment.

It is known to treat viral eye infections with acyclovir but such treatment is not entirely satisfactory. Topical acyclovir must be applied frequently and causes irritation of the eye. Oral acyclovir causes significant adverse side effects. Other antiviral medications such as gancyclovir, valacyclovir and valgancyclovir are used to treat viral eye infections, and such treatments are also not entirely satisfactory.

Gancyclovir is administered intravenously, and therefore cannot be used outside e.g. a hospital setting. Oral antiviral medications such as valacyclovir and valgancyclovir have disadvantages; they are known to cause fever, rash, diarrhea, and hematologic effects (e.g., neutropenia, anemia, thrombocytopenia). In some cases neutropenia may respond to lowering the dose or using drugs that stimulate the production of neutrophils by the bone marrow as granulocyte colony- stimulating factor [G-CSF], or granulocyte-macrophage colony-stimulating factor [GM-CSF]. These toxic effects can be difficult to manage.

It would therefore be advantageous to provide a better pharmaceutical for use in treating viral eye infections in humans.

Various enzymatically active ribonucleases, including ranpirnase and other proteins that are highly homologous to it, are known to have antiviral activity, and to have activity against viruses in the *Herpesviridae* family (specifically including *Herpes simplex* virus types 1 and 2 and *Human cytomegalovirus*) and also against type 2 *adenovirus.* However, proteins are known to be highly irritating to the eye due to an intense inflammatory response mediated by T-cells. For this reason, although ranpirnase and other related proteins have been investigated for use against various viral infections, they have not been investigated for use against viral infections of the eye.

Despite the expectation that proteinaceous ranpirnase would cause irritation in the eye, irritation of topically applied ranpirnase in the eye was studied in a rabbit model. In this experiment, ranpirnase was demonstrated to be non-irritating as determined using the Globally Harmonized System of Classification Evaluation Criteria and the European Economic Community Ocular Evaluation Criteria. This was a remarkable result, because administration of a foreign protein to the eye can produce corneal irritation. As a result, ranpirnase and other proteins that are highly homologous to it are expected to be useful in treating viral diseases of the human eye.

### Brief Description of the Drawings

The invention will be better understood with reference to the following drawings, in which:
Fig. 1 shows the scale used for scoring ocular lesions observed in a rabbit that has undergone a Draize test;
Fig. 2 shows the results of a Draize test in which a ranpirnase solution was applied to the right eye of three rabbits;
Fig. 3 shows the results of the Draize test of Fig. 2 in which the left eye of each of the rabbits was untreated; and
Fig. 4 shows the European Economic Community Ocular Evaluation Criteria used to classify the ocular irritation caused by a test article in a Draize test.

### Detailed Description

The present invention provides an RNase of the RNase A superfamily for use in treating a viral infection of a human eye, wherein the viral infection is caused by an adenovirus, and wherein the RNase is ranpirnase. The invention is further defined as in claims 2-11.

Ranpirnase is a proteinaceous enzymatically active ribonuclease that is disclosed and claimed in U.S. Patent No. 5,559,212. U.S. Patents Nos. 5,728,805 and 8,518,399 disclose another proteinaceous enzymatically active ribonuclease that is highly homologous to ranpirnase, i.e., the RNase of SEQ ID NO:2 in U.S. 5,728,805, herein referred to as the "805 variant".

US 8,518,399 discloses that ranpirnase and the '805 ranpirnase variant have antiviral activity against *Herpesviridae* viruses, specifically including *Herpes simplex* types 1 and 2 and *Human cytomegalovirus.*

U.S. Patent Publication No. 2012/0121569 A1 also discloses methods of treating infections originating from *Herpesviridae* viruses using an RNase such as ranpirnase,

Further, international patent application WO 2015/148768 A2 discloses that ranpirnase has antiviral activity against a number of viruses, including type 2 *adenovirus.*

RNases of the RNase A superfamily are pyrimidine-specific endonucleases found in high quantity in the pancreas of certain mammals and of some reptiles. They are involved in endonucleolytic cleavage of 3'-phosphomononucleotides and 3'- phosphooligonucleotides ending in C-P or U-P with 2',3'-cyclic phosphate intermediates. Members of this superfamily include ranpirnase and variants thereof, amphinase, r-Amphinase-2, bovine seminal vesicle and brain ribonucleases; kidney non-secretory ribonucleases; liver-type ribonucleases, angiogenin; eosinophil cationic protein, and pancreatic ribonucleases from different species including human and bovine pancreatic ribonucleases.

Ranpirnase is an RNase isolated from oocytes of the leopard frog *Rana pipiens* which is disclosed in U.S. Pat. No. 5,559,212, and was formerly known as ONCONASE^{®}. The amino acid sequence of ranpirnase is provided in SEQ ID NO: 1. Ranpirnase has been tested and found to be cytotoxic to cancer cells because of its enzymatic activity against RNA.

A variant of ranpirnase is disclosed in U.S. Pat. No. 5,728,805 (hereinafter, the "'805 variant"). The '805 variant is also an RNase, and has likewise been found to be cytotoxic to certain cancer cells. The '805 variant is a close variant of ranpirnase; its amino acid sequence is identical to that of ranpirnase except that it has valine instead of isoleucine at position 11, asparagine instead of aspartic acid at position 20, and arginine instead of serine at position 103 of the ranpirnase amino acid sequence. In some embodiments, the '805 variant is referred to as "Val11, Asn20, Arg103- Ranpirnase". The amino acid sequence of the '805 variant is provided in SEQ ID NO:2.

The '805 variant discussed above may be considered as a functional derivative of ranpirnase, because it comprises three amino acid substitutions compared to the ranpirnase amino acid sequence, but still has RNase activity.

In one embodiment the term "RNase of the RNase A superfamily" also includes fusion proteins of the RNase with another protein such as an antibody or antibody fragment. Such a fusion protein is described in WO 2005/080586 A1. U.S. Patent Publication No. 2012/0149085 A1 also discloses fusion proteins containing a recombinant RNase, such as ranpirnase. In an alternative embodiment the term "RNase of the RNase A superfamily" does not include such fusion proteins, meaning that the RNase is the only active protein which is used for the treatment of an adenoviral infection of the eye.

Reference is also made to U.S. Patent. No. 8,663,964 and U.S. Patent Publication Nos. 2012-0003266 and 2014-0037610 .

As discussed above, it has been found that an RNase of the RNase A superfamily, ranpirnase, can be used in the treatment of an adenoviral disease of the eye. "Viral diseases of the eye" are diseases which show symptoms predominantly in the eye of a subject and which are caused by viruses and not by bacteria. Symptoms of eye diseases include itching, excessive watering, red or pink color of the conjunctiva, pain, dry eyes, light sensitivity, swollen eyes, eye discharge and blurry vision. Viral diseases of the eye that can be treated include conjunctivitis keratoconjunctivitis and pharyngoconjuctival fever.

Viral conjunctivitis, also known as pink eye, is characterized by inflammation of the outermost layer of the white part of the eye and the inner surface of the eyelid. It makes the eye appear pink or reddish. There may also be pain, burning, scratchiness, or itchiness. Viral conjunctivitis is typically caused by adenovirus.

Adenoviridae are double-stranded DNA viruses which are classified in Baltimore class I. A adenovirus types 8, 19, 29, and 37 can cause epidemic keratoconjunctivitis, and *adenovirus* types 3, 4, and 7 can cause pharyngoconjuctival fever types 8, 19, 29, and 37 can cause epidemic keratoconjunctivitis, and *adenovirus* types 3, 4, and 7 can cause pharyngoconjuctival fever.

The terms "treating" and "treatment", as used herein, refer to administering to a subject having an adenoviral infection of the eye a therapeutically effective amount of an RNase, wherein the RNase is ranpirnase, or the ranpirnase '805 variant. As used herein, the term "treating" covers any treatment of an adenoviral infection of the eye which results in a desired pharmacologic and/or physiologic effect, including arresting disease development, causing regression of the disease, limiting spread of the adenovirus from one cell to another within an individual, limiting replication of an adenovirus in an individual, limiting entry of an adenovirus into the cell of an individual and reducing the number of viruses in an individual or a tissue of this individual.

The term "therapeutically effective amount" is used interchangeably herein with the term "therapeutically effective dose" and refers to an amount of an RNase that results in an improvement or remediation of the symptoms of a disease or condition to be treated. A therapeutically effective amount of an RNase, i.e., ranpirnase, or its '805 variant, in one embodiment, delays or minimizes the onset of, or hastens or increases recovery of a subject from, an adenoviral infection of the eye in a subject. In one embodiment, the RNase reduces the adenoviral titer in the eye of the infected subject. In another embodiment, the RNase prevents the adenoviral titer in the eye of the infected subject from increasing. In one embodiment, a therapeutically effective amount of an RNase provides a therapeutic benefit in the treatment or management of an adenoviral infection of the eye. In one embodiment, a therapeutically effective amount of an RNase reduces the spread of the adenovirus from one cell to another. A therapeutically effective amount may also prevent disease and/or reduce the severity of symptoms.

A therapeutically effective amount can be determined by the skilled person as a matter of routine experimentation. The therapeutically effective dosage of the pharmaceutical composition can be determined readily by the skilled artisan, for example, from animal studies. In addition, human clinical studies can be performed to determine the preferred effective dose for humans by a skilled artisan. Such clinical studies are routine and well known in the art. The precise dose to be employed will also depend on the route of administration. Effective doses may be extrapolated from dose-response curves derived from in vitro or animal test systems. The RNase may be administered to a subject in need thereof in a single dose or in multiple doses. In one embodiment, the RNase is administered to a subject in need thereof once per day, or in multiple doses per day. In one embodiment, the RNase is administered to the subject until symptoms resolve and/or until the subject is no longer at risk of an adenovirus infection.

In some embodiments, the administration of a therapeutically effective amount of the RNase reduces the adenovirus titer in the eye compared to a control not treated with the RNase, but infected with the adenovirus by at least 10%, preferably by at least 15%, or preferably by at least 20% and most preferably by at least 25%.

In some embodiments, the administration of a therapeutically effective amount of the RNase leads to a reduction of the adenovirus titer below the detection level. Determination of virus titers is for example discussed in Reischl (1996) Front Biosci. 1:e 72-7, Application of molecular biology-based methods to the diagnosis of infectious diseases.

In some embodiments, the RNase may be administered within 6 hours after the first symptoms of adenoviral infection of the eye have become apparent. In other embodiments, the RNase may be administered within 8, 10, 12, 15, 18 or 24 hours after the first symptoms of adenoviral infection of the eye become apparent or within two, three, four or five days after the first symptoms of adenoviral infection of the eye become apparent.

In some embodiments, multiple doses of the RNase are administered. The frequency of administration of these multiple doses may vary, depending on factors such as the severity of symptoms. For example, the RNase may be administered once per week, twice per week, three times per week, four times per week, every other day, once per day, twice per day or three times a day.

The duration of the administration of the RNase i.e. the period over which the RNase is administered, can vary depending on factors such as the severity of symptoms, patient response, etc. For example, the RNase can be administered over a period ranging from one day, three days, seven days, two weeks, four weeks, two months, three months, four months, five months or six months or longer.

The RNase or pharmaceutical composition comprising the RNase is preferably administered topically to the eye, meaning that the RNase or pharmaceutical composition comprising RNase is administered directly to the eye and not to another place of the body. The topical administration may be by eye drops, a suspension, an emulsion, an ointment, a solution, a gel, liposomes, nanoparticles, microemulsions, nanoemulsions, nanosuspensions, niosomes, dendrimers and hydrogels.

The site of action of ranpirnase administered topically may be different layers of the cornea, conjunctiva, sclera, and the other tissues of the anterior segment such as the iris and ciliary body (anterior uvea).

Alternatively, the RNase or pharmaceutical composition comprising the RNase may be administered by the intravitreal, intracameral, subconjunctival, subtenon, retrobulbar or posterior juxtascleral route.

The pharmaceutical composition containing the RNase has to be sterile to be administered to the eye.

Suitable excipients for ophthalmic pharmaceutical compositions include preservatives such as benzalkonium chloride, polyquaternium-1 (Polyquad), sodium perborate, oxychloro-complex (Purite^{®}), chlorobutanol, benzethonium chloride, cetyl pyridinium chloride, benzyl bromide, EDTA, polyaminopropyl biguanide, phenylmercury nitrate, phenylmercury acetate, thimerosal, merthiolate, acetate and phenylmercury borate, polymyxin B sulphate, chlorhexidine, methyl and propyl parabens, phenylethyl alcohol, quaternary ammonium chloride, sodium benzoate, sodium propionate, sorbic acid and SofZia.

Further excipients in the pharmaceutical composition to be administered to the eye may be used to control the viscosity of the composition and include povidone, polyvinyl alcohol, polyvinylpyrrolidone, methylcellulose, hydroxypropyl methylcellulose, hydroxyethylcellulose and carboxymethyl cellulose.

Surfactants may be added to the pharmaceutical composition for dispersing insoluble ingredients or to aid in solubilization. Preferably, non-ionic surfactants such as polysorbates including polysorbate 80 or polysorbate 20 are used. Other suitable surfactants include polyoxyl 40 stearate and polyethylene glycol.

Usually, the pharmaceutical composition also contains an ingredient to adjust the pH of the composition and to buffer within a certain pH range. The pharmaceutical composition may have a pH between 5.0 and 7.5. A boric acid vehicle or Sorensen's modified phosphate buffer may be used as buffer.

Further, a tonicity agent to adjust the tonicity of the composition can be used. Suitable tonicity-adjusting ingredients include sodium chloride, sodium nitrate, sodium sulfate, potassium chloride, dextrose, glycerol, propylene glycol and mannitol.

The term "pharmaceutical composition" as used herein encompasses a composition suitable for administration to a human subject. In general a "pharmaceutical composition" is sterile, and free of contaminants that are capable of eliciting an undesirable response within the subject.

The term "pharmaceutically acceptable carrier" as used herein includes any and all solvents, dispersion media, coatings, antibacterial and antifungal agents, isotonic and absorption delaying agents and the like. The use of such media and agents for pharmaceutically active substances is well known in the art. These agents are generally safe, non-toxic and neither biologically nor otherwise undesirable.

Supplementary active ingredients also can be incorporated into the pharmaceutical compositions. The RNase may be administered together with other biologically active agents. Alternatively, the RNase is the only biologically active agent in the pharmaceutical composition.

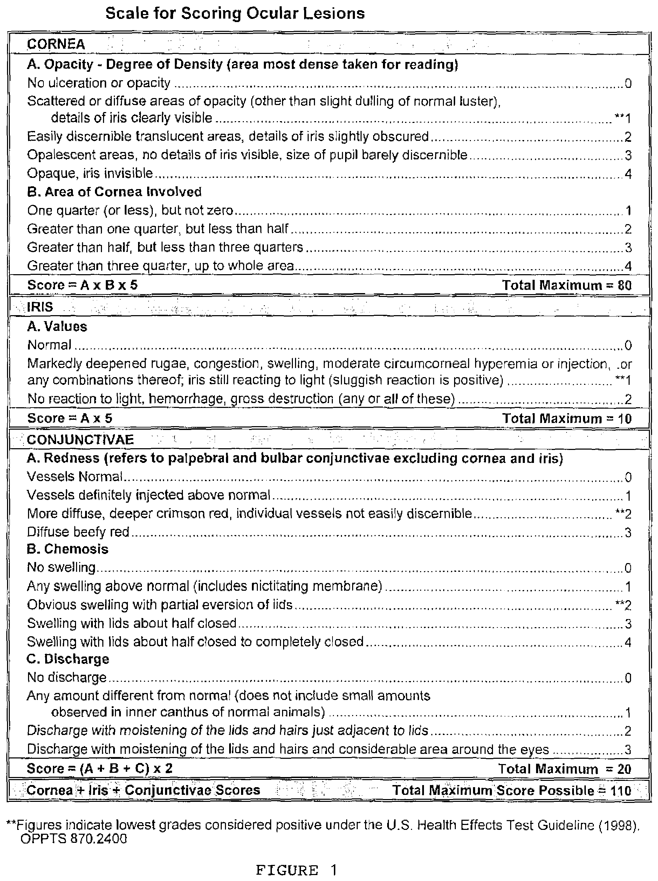

It is possible to determine whether an RNase is non-irritating to the eye in a Draize test using the Globally Harmonized System of Classification Evaluation Criteria and using the European Economic Community Ocular Evaluation Criteria. In the Draize test the RNase is placed in the conjunctival sac of a rabbit's eye and the eye is examined at 1, 24, 48 and 72 hours after instillation of ranpirnase. The criteria evaluated are corneal opacity, iris lesion, conjunctival redness and conjunctival edema. If the score for each of these criteria is zero, the RNase is considered non- irritating to the eye. Further information on the evaluation can be taken from Figures 1 to 4.

As stated above, before the present invention, no person of ordinary skill in the art would administer ranpirnase to the eye. However, such administration of ranpirnase has been modeled using the Draize test and the results of this experiment demonstrate that ranpirnase is non-irritating as defined by two accepted standards.

### Example

A 0.1 % mL solution made up of 0.1 % ranpirnase in a proprietary aqueous solution used as a vehicle was used as a test article. Three rabbits were used; each was a male New Zealand White rabbit that was approximately 16 weeks old at the time of the experiment and that weighed 3.3 to 3.4 kg.

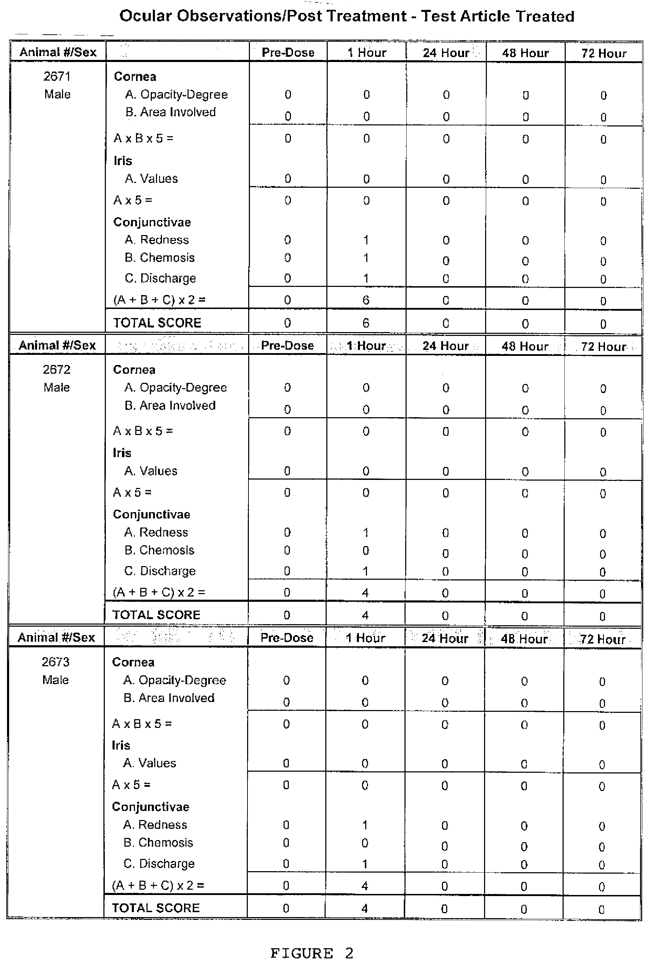

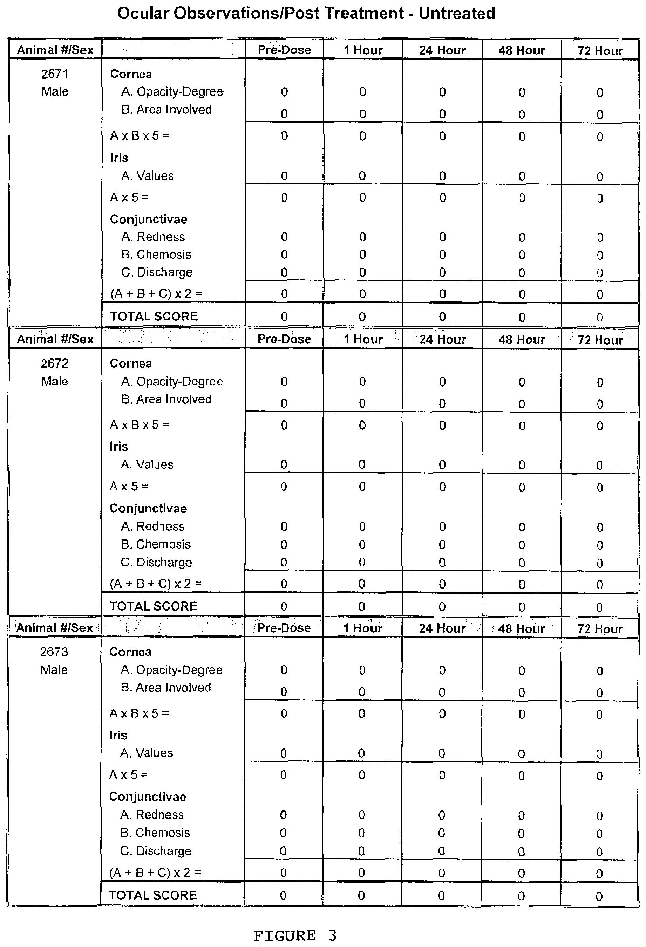

After administration of two drops of Tetracaine pre-anesthetic to the corneal surface of both eyes of each rabbit, the test article was placed in the conjunctival sac of the right eye of each rabbit by gently pulling the lower lid away from the eyeball; the lids were gently held together for approximately one second to limit the loss of the test material. The left eye of each rabbit remained untreated and served as the control. The eyes of the animals were examined at 1 (+ 15 minutes), 24, 48, and 72 hours (+ 1 hour) after installation of the test article. The grades of ocular reaction according to Draize (Fig. 1) were manually recorded at each examination (Figs. 2 and 3). As can be seen in Fig. 2, there was an ocular reaction in each animal one hour post- instillation of the test article but in every instance that reaction was completely resolved by 24 hours and thereafter.

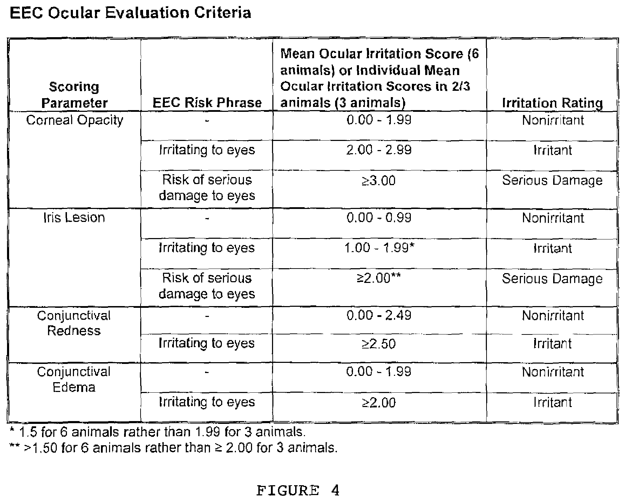

To determine the degree of irritation caused by the test article using the European Economic Community Ocular Evaluation Criteria (Fig. 4), the total ocular irritation scores for the examinations at 24, 48, and 72 hours were individually added for corneal opacity, iris lesion, conjunctival redness, and conjunctival edema and the mean scores for these scoring parameters were compared to the European Economic Community Ocular Evaluation Criteria. Because all these scores (and therefore the calculated mean scores) were zero, the test subject was considered to be non- irritating as defined by the European Economic Community Ocular Evaluation Criteria.

To determine the degree of irritation caused by the test article using the Globally Harmonized System of Classification Evaluation Criteria, the 24-, 48-, and 72-hour scores were added separately for each animal and each total divided by 3 (three time points) to yield the individual mean scores for each animal. Because all these scores (and therefore the calculated quotients) were zero, the test subject was considered to be non-irritating as defined by the Globally Harmonized System of Classification Evaluation Criteria.

Hence, these test data demonstrate a new and unexpected result: ranpirnase delivered to the eye in an aqueous solution is non-irritating as defined by the Globally Harmonized System of Classification Evaluation Criteria and by the European Economic Community Ocular Evaluation Criteria, even though ranpirnase is a protein (which would be expected to be irritating to the eye).

Although this experiment was carried out using a solution of ranpirnase in a proprietary aqueous vehicle, a person of ordinary skill in the art would consider it likely that solutions of the ranpirnase '805 variant would behave in the same way because of its similarities to ranpirnase in respect of activity and homology.

## Claims

1. RNase of the RNase A superfamily for use in treating a viral infection of a human eye, wherein the viral infection is caused by an adenovirus, and wherein the RNase is ranpirnase.

2. The RNase for use according to claim 1, wherein the ranpirnase is a fusion protein comprising ranpirnase.

3. The RNase for use according to any of claims 1 or 2, wherein the ranpirnase is SEQ ID NO: 1 or SEQ ID NO: 2.

4. The RNase for use according to any of claims 1-3, wherein the adenoviral infection is the cause of viral conjunctivitis, epidemic keratoconjunctivitis, or pharyngoconjuctival fever.

5. The RNase for use according to claim 1, wherein the adenovirus is an adenovirus 3, an adenovirus 4, an adenovirus 7, an adenovirus 8, an adenovirus 19, an adenovirus 29 or an adenovirus 37.

6. The RNase for use according to any of claims 1-5, wherein the RNase of the RNase A superfamily is topically administered to the eye.

7. The RNase for use according to any of claims 1-6, wherein the RNase of the RNase A superfamily is formulated as an ophthalmic pharmaceutical composition.

8. The RNase for use according to claim 7, wherein the ophthalmic pharmaceutical composition is a solution, a suspension, a nanosuspension, an emulsion, a microemulsion, a nanoemulsion, an ointment, a gel, a hydrogel, liposomes, niosomes, nanoparticles, or dendrimers.

9. The RNase for use according to claim 7 or claim 8, wherein the ophthalmic pharmaceutical composition further comprises a pharmaceutically acceptable carrier and/or a pharmaceutically acceptable excipient.

10. The RNase for use according to any of claims 1-7, wherein the RNase is formulated for administration as eye drops.

11. The RNase for use according to any of claims 1-10, wherein the RNase is non-irritating to the eye as determined using
a. the Globally Harmonized System of Classification Evaluation Criteria and
b. the European Economic Community Ocular Evaluation Criteria.

## Patentansprüche

1. Eine RNase aus der RNase-A-Superfamilie zur Verwendung bei der Behandlung einer viralen Infektion des menschlichen Auges, wobei die virale Infektion durch ein Adenovirus verursacht ist und wobei die RNase Ranpirnase ist.

2. Die RNase zur Verwendung gemäß Anspruch 1, wobei die Ranpirnase ein Fusionsprotein umfassend Ranpirnase ist.

3. Die RNase zur Verwendung gemäß einem der Ansprüche 1 oder 2, wobei die Ranpirnase SEQ ID NR: 1 oder SEQ ID NR: 2 ist.

4. Die RNase zur Verwendung gemäß einem der Ansprüche 1 bis 3, wobei die Adenovirus-Infektion virale Konjunktivitis, epidemische Keratokonjunktivitis oder pharyngokonjunktivales Fieber verursacht.

5. Die RNase zur Verwendung gemäß Anspruch 1, wobei das Adenovirus ein Adenovirus 3, 4, 7, 8, 19, 29 oder 37 ist.

6. Die RNase zur Verwendung gemäß einem der Ansprüche 1 bis 5, wobei die RNase aus der RNase-A-Superfamilie topisch am Auge verabreicht wird.

7. Die RNase zur Verwendung gemäß einem der Ansprüche 1 bis 6, wobei die RNase aus der RNase-A-Superfamilie als ophthalmische pharmazeutische Zusammensetzung formuliert ist.

8. Die RNase zur Verwendung gemäß Anspruch 7, wobei die ophthalmische pharmazeutische Zusammensetzung eine Lösung, Suspension, Nanosuspension, Emulsion, Mikroemulsion, Nanoemulsion, Salbe, Gel, Hydrogel, Liposomen, Niosomen, Nanopartikel oder Dendrimere ist.

9. Die RNase zur Verwendung gemäß Anspruch 7 oder 8, wobei die ophthalmische pharmazeutische Zusammensetzung ferner einen pharmazeutisch akzeptablen Träger und/oder ein pharmazeutisch akzeptables Hilfsmittel umfasst.

10. Die RNase zur Verwendung gemäß einem der Ansprüche 1 bis 7, wobei die RNase zur Verabreichung als Augentropfen formuliert ist.

11. Die RNase zur Verwendung gemäß einem der Ansprüche 1 bis 10, wobei die RNase nachweislich nicht reizend auf das Auge ist, bestimmt anhand:
a. der Klassifizierungskriterien des Global Harmonized System (GHS) und
b. der Bewertungskriterien der Europäischen Wirtschaftsgemeinschaft für okuläre Irritation.

## Revendications

1. RNase de la superfamille RNase A pour utilisation dans le traitement d'une infection virale d'un œil humain, où l'infection virale est causée par un adénovirus, et où la RNase est la ranpirnase.

2. RNase pour utilisation selon la revendication 1, où la ranpirnase est une protéine de fusion comprenant la ranpirnase.

3. RNase pour utilisation selon l'une quelconque des revendications 1 ou 2, où la ranpirnase est SEQ ID NO : 1 ou SEQ ID NO : 2.

4. RNase pour utilisation selon l'une quelconque des revendications 1 à 3, où l'infection adénovirale est la cause d'une conjonctivite virale, d'une kératoconjonctivite épidémique ou d'une fièvre pharyngoconjonctivale.

5. RNase pour utilisation selon la revendication 1, où l'adénovirus est un adénovirus 3, un adénovirus 4, un adénovirus 7, un adénovirus 8, un adénovirus 19, un adénovirus 29 ou un adénovirus 37.

6. RNase pour utilisation selon l'une quelconque des revendications 1 à 5, où la RNase de la superfamille RNase A est administrée par voie topique à l'œil.

7. RNase pour utilisation selon l'une quelconque des revendications 1 à 6, où la RNase de la superfamille RNase A est formulée sous la forme d'une composition pharmaceutique ophtalmique.

8. RNase pour utilisation selon la revendication 7, où la composition pharmaceutique ophtalmique est une solution, une suspension, une nanosuspension, une émulsion, une microémulsion, une nanoémulsion, une pommade, un gel, un hydrogel, des liposomes, des niosomes, des nanoparticules ou des dendrimères.

9. RNase pour utilisation selon la revendication 7 ou la revendication 8, où la composition pharmaceutique ophtalmique comprend en outre un véhicule pharmaceutiquement acceptable et/ou un excipient pharmaceutiquement acceptable.

10. RNase pour utilisation selon l'une quelconque des revendications 1 à 7, où la RNase est formulée pour être administrée sous forme de collyre.

11. RNase pour utilisation selon l'une quelconque des revendications 1 à 10, où la RNase n'est pas irritante pour les yeux, tel que déterminé en utilisant
a. le Système général harmonisé de classification des critères d'évaluation et
b. les critères d'évaluation oculaire de la Communauté économique européenne.
